# EUROPÄISCHE PATENTSCHRIFT

(11) **EP 0 747 699 B1**
(45) Veröffentlichungstag und Bekanntmachung des Hinweises auf die Patenterteilung: **09.12.1998**
(21) Anmeldenummer: 96108625.3
(22) Anmeldetag: 30.05.1996
(51) Int. Cl.: G01N 33/53, G01N 33/78, G01N 33/543

(54) **Entstörungsreagenz für die Bestimmung eines Analyten mit einem lumineszenzfähigen Metallkomplex**
Reagent for suppressing interference for use in the measurement of an analyte using a luminescent metal complex
Réactif pour la suppression des interactions pour utilisation dans la détermination d'une analyte utilisant des complexes métalliques de marquage luminescent

(30) Priorität: 31.05.1995 DE 19519973
(43) Veröffentlichungstag der Anmeldung: 11.12.1996
(73) Patentinhaber: BOEHRINGER MANNHEIM GMBH, 68305 Mannheim (DE)
(72) Erfinder: Eckert, Bernhard, Dr., 82362 Weilheim (DE); Lenz, Helmut, Dr., 82327 Tutzing (DE); Franken, Norbert, Dr., 82319 Starnberg (DE); Josel, Hans-Peter, Dr., 82362 Weilheim (DE); Ofenloch-Hähnle, Beatus, Dr., 82398 Polling (DE)
(74) Vertreter: Weiss, Wolfgang, Dipl.-Chem. Dr.

(56) Entgegenhaltungen:
- EP-A- 0 309 230
- WO-A-93/12424
- US-A- 4 810 635
- US-A- 5 221 605

## Beschreibung

Die vorliegende Erfindung betrifft ein Verfahren zur Bestimmung eines Analyten in einer Probeflüssigkeit mit einem lumineszenzfähigen Metallkomplex als Analyt-spezifischer Markierungsgruppe zur Erzeugung eines Meßsignals sowie einen Reagenzienkit zur Durchführung dieses Verfahrens.

Die Verwendung von lumineszenzfähigen Metallkomplexen zum Nachweis von Analyten in Probeflüssigkeiten, insbesondere in Körperflüssigkeiten, wie etwa Humanseren, ist bekannt. EP-A-0 178 450 offenbart z. B. Rutheniumkomplexe, die an ein immunologisch aktives Material gekoppelt sind, wobei die Rutheniumkomplexe drei gleiche oder verschiedene bi- oder polycyclische Liganden mit mindestens zwei stickstoffhaltigen Heterocyclen enthalten. EP-A-0 580 979 offenbart die Verwendung von Osmium- oder Rutheniumkomplexen als Markierungsgruppen für die Elektrochemilumineszenz. Als Liganden für diese Komplexe werden stickstoffhaltige Heterocyclen, beispielsweise Bipyridine, genannt. WO 87/06706 offenbart weitere Metallkomplexe, die sich als Markierungsgruppen für Elektrochemilumineszenzmessungen eignen.

Für Elektrochemilumineszenzmessungen geeignete Metallkomplexe werden auch in DE 44 30 998.8, DE 44 39 345.8, DE 44 39 346.6 und DE 44 39 347.4 offenbart.

Ein Nachteil bei der Verwendung von lumineszenzfähigen Metallkomplexen als Markierungsgruppen bei der Bestimmung von Analyten besteht darin, daß in bestimmten Proben, insbesondere in Serumproben, erhebliche Störungen auftreten können. Diese Störungen treten in verschiedenen Testformaten, insbesondere in kompetitiven Assays in einer solchen Häufigkeit auf, die eine erhebliche Beeinträchtigung für die routinemäßige diagnostische Anwendbarkeit darstellen kann. Die Folge dieser Störungen ist ein Signalquenching, d. h. das gemessene Signal ist gering und je nach Testformat werden falsch positive oder negative Analyseergebnisse erhalten.

Aus dem Stand der Technik ist bekannt, daß Bestandteile in Seren vorkommen, die mit immunologischen Reagenzien reagieren und durch Vernetzung oder Veränderung der unspezifischen Bindung eine analytunabhängige Modulation des Meßsignals bewirken können. Dies kann zu falscher Wiederfindung bei der Analytenbestimmung führen. Störungen dieser Art können üblicherweise durch Zusatz einer immunologischen Komponente beseitigt werden, die mit störenden Serumbestandteilen bevorzugt reagiert und dadurch eine Inaktivierung herbeiführt. Derartige Entstörungsreagenzien erwiesen sich für die Beseitigung der oben genannten Störungen jedoch als nicht erfolgreich.

US-A-4,810,635 beschreibt die Entstörung eines homogenen Apoenzym-Reaktivierungsimmunoassays mit einer Flavinadenindinukleotid (FAD) -Markierungsgruppe, wobei ein strukturelles Analogon von FAD, z. B. Flavinmononocleotid (FMN) oder Riboflavin, als Entstörungsreagenz, zugesetzt wird. Ein Entstörungsreagenz für Metallkomplex-Markierungsgruppen, das insbesondere für heterogene Testformate geeignet ist, wird weder offenbart noch nahegelegt.

Die der vorliegenden Erfindung zugrundeliegende Aufgabe bestand somit darin, ein Entstörungsreagenz für Testverfahren zur Bestimmung von Analyten mit lumineszenzfähigen Metallkomplexen als Markierungsgruppen bereitzustellen, welches einerseits eine zuverlässige Entstörung von Störseren erlaubt und andererseits die Genauigkeit und Sensitivität des Tests nicht signifikant beeinträchtigt.

Diese Aufgabe wird gelöst durch ein Verfahren zur Bestimmung eines Analyten in einer Probeflüssigkeit mit einem lumineszenzfähigen Metallkomplex als Analyt-spezifischer Markierungsgruppe zur Erzeugung eines Meßsignals, wobei man weiterhin einen unspezifischen Metallkomplex als Entstörungsreagenz zusetzt, der eine mit der Markierungsgruppe chemisch verwandte Struktur besitzt.

Im erfindungsgemäßen Verfahren zur Bestimmung eines Analyten wird zur Erzeugung eines Meßsignals ein lumineszenzfähiger Metallkomplex als Analyt-spezifische Markierungsgruppe verwendet. Dies bedeutet, daß ein lumineszenzfähiger Metallkomplex, wie im folgenden definiert, direkt oder indirekt an eine Analyt-spezifische Testkomponente gekoppelt ist und als Markierungsgruppe zum spezifischen Nachweis des Analyten dient. Bei einer direkten Kopplung ist der Metallkomplex kovalent mit einer Analyt-spezifischen Testkomponente, z. B. einen mit dem Analyten bindefähigen Rezeptor oder einem Analogon des Analyten verbunden. Bei einer indirekten Kopplung ist der Metallkomplex z. B. kovalent an eine Testkomponente gebunden, die selbst nicht Analyt-spezifisch ist, sondern mit einer Analyt-spezifischen Testkomponente reagieren kann.

Der als Markierungsgruppe verwendete Metallkomplex kann eine nachweisbare Lumineszenzreaktion erzeugen. Der Nachweis dieser Lumineszenzreaktion kann beispielsweise durch Fluoreszenz- oder durch Elektrochemilumineszenzmessung erfolgen. Vorzugsweise erfolgt der Nachweis durch Elektrochemilumineszenzmessung.

Das Metallkation des Komplexes ist ein zwei- oder dreiwertiges Metallkation, beispielsweise ein Übergangsmetall oder ein Seltenerdmetall. Vorzugsweise ist das Metall Ruthenium, Osmium, Rhenium, Iridium, Rhodium, Platin, Palladium oder Chrom. Besonders bevorzugt sind Ruthenium, Iridium, Rhenium, Chrom und Osmium. Am meisten bevorzugt ist Ruthenium.

Überraschenderweise wurde festgestellt, der Zusatz eines Analytunspezifischen Metallkomplexes, der eine mit der Markierungsgruppe chemisch verwandte Struktur besitzt, eine teilweise oder vollständige Beseitigung der durch Signalquenching hervorgerufenen Störungen ermöglicht und andererseits auch nicht zu Verfälschungen der Meßergebnisse führt. Dieser unspezifische Komplex ist in seiner chemischen Struktur der verwendeten Markierungsgruppe ähnlich, wobei sich diese Ähnlichkeit auf das Metall, die Komplexliganden und gegebenenfalls auf die zur Kopplung der Markierungsgruppe an Testkomponenten verwendeten Linkerstrukturen sowie Trägermoleküle beziehen kann.

Vorzugsweise wird der unspezifische Metallkomplex ausgewählt aus Übergangsmetall- und Seltenerdmetallkomplexen, insbesondere aus Ruthenium-, Rhodium-, Osmium-, Nickel-, Eisen-, Kobalt-, Iridium-, Palladium-, Platin-, Chrom- und Rheniumkomplexen sowie Kombinationen davon. Besonders bevorzugt ist der unspezifische Metallkomplex ausgewählt aus Rhodium-, Osmium-, Ruthenium- und Eisenkomplexen sowie Kombinationen davon. Am besten geeignet sind Osmium- und Eisenkomplexe, da sie eine besonders große strukturelle Ähnlichkeit zu den bevorzugt als Markierungsgruppen verwendeten Rutheniumkomplexen aufweisen. Eisenkomplexe sind bevorzugt, da sie bei einer Elektrochemilumineszenzmessung unter Testbedingungen kein signifikantes Signal erzeugen. Auch Rhodiumkomplexe sind bevorzugt, da sie unter Testbedingungen kein Elektrochemilumineszenzsignal erzeugen. Sie müssen jedoch zur wirksamen Beseitigung von Störungen in etwas größerer Menge zugesetzt werden.

In bestimmten Testvarianten sind bei Verwendung von Rutheniumkomplexen als Markierungsgruppen auch Ruthenium-Entstörungskomplexe geeignet, z. B. wenn Ruthenium an einen Peptidträger gebunden vorliegt. Insbesondere werden Osmium- und Rutheniumkomplexe, die unter den Testbedingungen ein Elektrochemilumineszenzsignal erzeugen können, in kompetitiven Assays als Entstörungsreagenzien eingesetzt. In Sandwich-Assays ist ihre Verwendung weniger bevorzugt.

Die Liganden der Markierungs- und Entstörungskomplexe sollen möglichst gleich sein, um eine optimale Entstörungswirkung zu erreichen. Beispiele für geeignete Liganden sind aromatische heterocyclische Polydentatliganden, z. B. stickstoffhaltige Heterocyclen. Bevorzugt sind Bipyridyl, Bipyrazyl, Terpyridyl und Phenanthrolyl. Besonders bevorzugt werden die Liganden aus gegebenenfalls substituierten Bipyridin- und Phenanthrolin-Ringsystemen ausgewählt.

Der unspezifische Entstörungskomplex kann als Komplex alleine, als Komplex mit Linker oder/und als trägergebundener Komples zugesetzt werden. Da verschiedene Störseren unterschiedlich auf verschiedene Entstörderivate reagieren können, kann es oft von Vorteil sein, Kombinationen von verschiedenen Entstörderivaten zu verwenden.

Beispiele für geeignete Träger sind Substanzen, die nicht mit der Bestimmung des Analyten interferieren, d. h. eine unerwünschte Bindung mit Analyt-spezifischen Testkomponenten eingehen. Als Träger können grundsätzlich beliebige natürliche oder synthetische Substanzen, z. B. Polymere oder auch kleine Moleküle verwendet werden, sofern sie mit den übrigen Testkomponenten kompatibel sind. Spezifische Beispiele für geeignete Träger sind Aminosäuren, Peptide, Proteine und Polypeptide, die gegebenenfalls vernetzt sein können, Nukleotide, Nukleinsäuren, synthetische Träger, z. B. Dextrane oder Nukleinsäureanaloga wie etwa PNA (peptidische Nukleinsäuren), Kohlenhydrate oder Steroide. Beispiele für geeignete Polypeptide sind Albumine, z. B. Rinderserumalbumine, unspezifische Immunglobuline, Immunglobulinfragmente, Beta-Galaktosidase und Polylysin. Bevorzugte Träger sind Nukleinsäuren, Nukleinsäureanaloga, Peptide oder/und Polypeptide. Besonders bevorzugt werden trägergebundene Komplexe verwendet, die eine Stöchiometrie von Komplex zu Träger von größer oder gleich 1 : 1 aufweisen, d. h. die statistisch mindestens einen Metallkomplex pro Trägermolekül enthalten.

Wenn der Komplex gekoppelt an einen Träger oder Linker eingesetzt wird, kann es bevorzugt sein, daß verschiedene Komplexe gekoppelt an ein einziges Linker- bzw. Trägermolekül oder gekoppelt an mehrere gleichartige Linker- bzw. Trägermoleküle verwendet werden. Andererseits können gleiche Komplexe auch gekoppelt an verschiedene Linker- bzw. Träger oder verschiedene Komplexe gekoppelt an verschiedene Linker bzw. Träger eingesetzt werden. Auch wenn die Komplexe alleine eingesetzt werden, kann man mehrere verschiedene Komplexe, z. B. Komplexe mit gleichen Metallkationen und verschiedenen Liganden, Komplexe mit verschiedenen Metallkationen und gleichen Liganden oder Komplexe mit verschiedenen Metallkationen und verschiedenen Liganden verwenden.

Bei der Durchführung des erfindungsgemäßen Verfahrens ist es weiterhin bevorzugt, daß der unspezifische Metallkomplex in einem stöchiometrischen Überschuß bezüglich der Analyt-spezifischen Markierungsgruppe eingesetzt wird, z. B. in einem Überschuß von bis zu 10⁶ : 1, insbesondere bis zu 10⁵ : 1.

Die Herstellung der erfindungsgemäßen Entstörungskomplexe kann durch Reaktion eines Metallsalzes, z. B. eines Metallhalogenids mit den entsprechenden Liganden und gegebenenfalls anschließenden Austausch des Halogenidions durch Hexafluorophosphat-, Trifluoracetat- oder Tetrafluoroborat-Anionen erfolgen. Derartige Verfahren sind im Stand der Technik, z. B. in EP-A-0 178 450 und EP-A-0 255 534 beschrieben. Auf diese Offenbarung wird hiermit Bezug genommen.

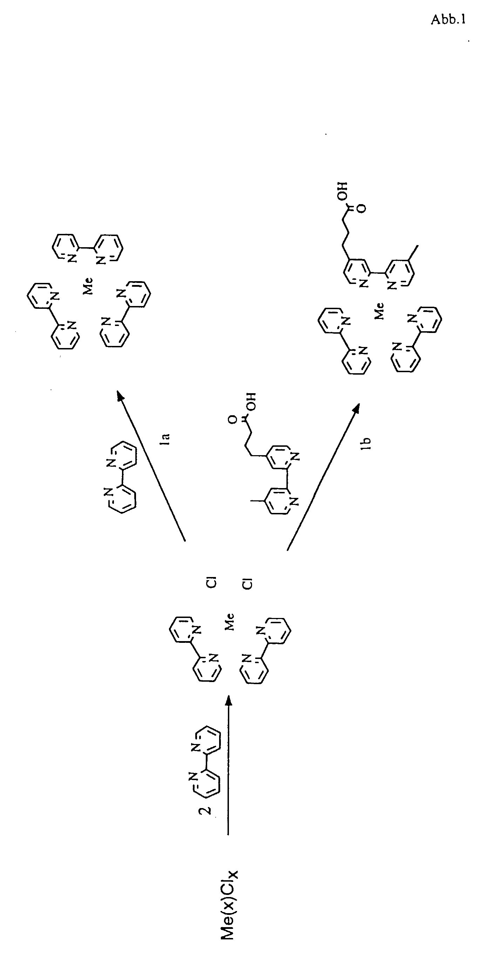

Vorzugsweise werden die Metallhalogenide der gewünschten Komplexe in einem geeigneten Lösungsmittel, z. B. Wasser/Ethanol oder Dimethylformamid, bei Raumtemperatur oder unter Rückfluß mit zwei Äquivalenten eines Polyaza-Bis-Heterozyklus, z. B. 2,2'-Bipyridin oder Phenanthrolin umgesetzt. Dieser Komplex wird dann mit einem weiteren Äquivalent des gleichen oder eines anderen Liganden, der gegebenenfalls eine Linkerstruktur enthalten kann, erneut umgesetzt. Ein entsprechendes Reaktionsschema ist in Abb. 1 gezeigt. Die gemäß Reaktionsweg 1a erhaltenen Komplexe ohne Linker können direkt als Entstörungsreagenz eingesetzt werden. Die gemäß Reaktionsweg 1b erhaltenen Komplexe mit Linker können ebenfalls direkt als Entstörungsreagenz eingesetzt oder an einem Träger gekoppelt werden.

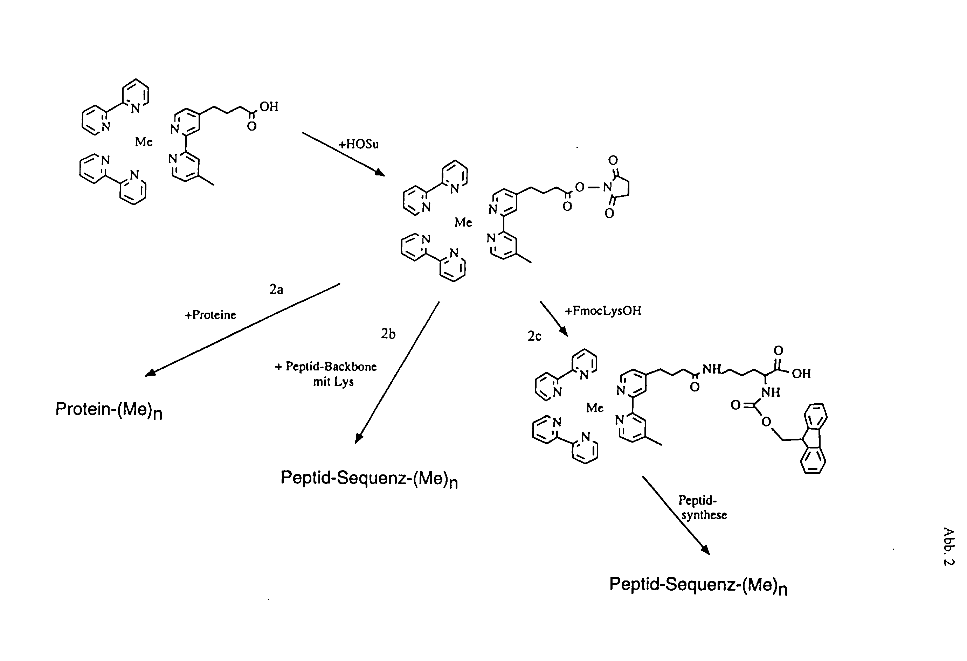

In Abb. 2 sind mehrere Möglichkeiten der Kopplung an Träger dargestellt. Hierzu wird zunächst eine reaktive Gruppe, z. B. eine aktivierte Carbonsäuregruppe, wie etwa ein Carbonsäurehalogenid, Carbonsäureanhydrid oder ein Aktivester, z. B. ein N-Hydroxysuccinimidester, ein Maleimid oder eine photoaktivierbare Gruppe, z. B. ein Azid in den Komplex eingeführt. Gemäß den Reaktionswegen 2a und 2b kann der aktivierte Komplex anschließend mit Trägermolekülen, z. B. Proteinen oder Peptid-Backbones umgesetzt werden. Andererseits können Metallkomplex-Peptidkonjugate auch durch eine Festphasen-Peptidsynthese aus entsprechenden geschützten Aminosäurederivaten (z. B. FMOC-Derivate) gemäß den in DE-44 30 998.8 und DE-44 39 345.8 beschriebenen Verfahren hergestellt werden. Auf diese Offenbarung wird hiermit Bezug genommen.

Der Nachweis des Metallkomplexes im erfindungsgemäßen Verfahren erfolgt vorzugsweise durch Elektrochemilumineszenz, wobei lumineszierende Spezies elektrochemisch an der Oberfläche einer Elektrode erzeugt werden. Beispiele zur Durchführung von Elektrochemilumineszenz-Assays mit Metallkomplexen des Standes der Technik finden sich in EP-A-0 580 979, WO 90/05301, WO 90/11511 und WO 92/14138. Auf die dort offenbarten Verfahren und Vorrichtungen für Lumineszenzassays wird hiermit Bezug genommen. Die Elektrochemilumineszenzassays werden vorzugsweise als heterogener Assay in Gegenwart einer Festphase durchgeführt, die besonders bevorzugt aus Mikropartikeln, insbesondere aus magnetischen Mikropartikeln, besteht, und mit einer reaktiven Beschichtung versehen ist, z. B. Streptavidin. Auf diese Weise können Immunkomplexe, die einen Metallkomplex als Markierungsgruppe enthalten, in einer an die Festphase gebundene Form nachgewiesen werden. Andererseits kann die Elektrochemilumineszenzmessung auch als homogener Assay durchgeführt werden (vgl. z. B. EP-A-0 199 804; EP-A-0 265 519).

Die Elektrochemilumineszenz-Messung wird vorzugsweise in Gegenwart eines Reduktionsmittels für den Metallkomplex durchgeführt, z. B. einem Amin. Bevorzugt sind aliphatische Amine, insbesondere primäre, sekundäre und tertiäre Alkylamine, deren Alkylgruppen jeweils 1 bis 3 Kohlenstoffatome aufweisen. Besonders bevorzugt ist Tripropylamin. Das Amin kann jedoch auch ein aromatisches Amin, wie Anilin, oder ein heterocyclisches Amin sein. Das Reduktionsmittel kann bereits in der Ligandensphäre des Komplexes integriert sein.

Weiterhin kann gegebenenfalls als Verstärker ein nicht-ionisches oberflächenaktives Mittel, z. B. ein ethoxyliertes Phenol bzw. ein Alkohol vorhanden sein. Derartige Substanzen sind beispielsweise unter den Bezeichnungen Triton-X 100, Triton-N 401 und Thesit erhältlich.

Andererseits kann der Nachweis des lumineszierenden Metallkomplexes auch durch Fluoreszenz erfolgen, wobei der Komplex durch Bestrahlung mit einem Licht der geeigneten Wellenlänge angeregt und die daraus resultierende Fluoreszenzstrahlung gemessen wird. Beispiele zur Durchführung von Fluoreszenz-Assays finden sich in EP-A-0 178 450 und EP-A-0 255 534. Auf diese Offenbarung wird hiermit Bezug genommen.

In einer bevorzugten Ausführungsform des erfindungsgemäßen Verfahrens erfolgt die Bestimmung des Analyten in einem Immunoassay. Beispiele für Analyten, die in einem Immunoassay bestimmt werden können, sind Antigene oder Haptene. Der Nachweis des Analyten erfolgt in diesem Fall durch Reaktion mit einem oder mehreren spezifischen Antikörpern. Andererseits kann ein Immunoassay auch zur Bestimmung von Immunglobulinen in einer Probelösung durch Reaktion mit spezifischen Antigenen durchgeführt werden. In einer weiteren bevorzugten Ausführungsform erfolgt die Bestimmung des Analyten in einem Nukleinsäure-Hybridisierungsassay. Diese Ausführungsform dient insbesondere zur Bestimmung von Nukleinsäureanalyten.

Beim erfindungsgemäßen Verfahren kann die Bestimmung des Analyten in einem Sandwich-Assay durchgeführt werden, z. B. in einem heterogenen Sandwich-Assay, wobei man
(a) die den Analyten enthaltende Probeflüssigkeit in Gegenwart einer reaktiven Festphase mit mindestens zwei Analyt-spezifischen Rezeptoren inkubiert, von denen einer an die Festphase gebunden oder in einer an die Festphase bindefähigen Form vorliegt und ein anderer die Metallkomplex-Markierungsgruppe trägt,
(b) die Festphase gegebenenfalls von der Inkubationsflüssigkeit abtrennt und
(c) das Vorhandensein oder/und die Menge des Analyten in der Festphase oder/und in der flüssigen Phase durch Bestimmung der Markierung nachweist.

Beispiele für Analyt-spezifische Rezeptoren sind Antigene, Haptene, Antikörper, Nukleinsäuren oder Nukleinsäureanaloga, die eine spezifische Immun- oder Hybridisierungsreaktion mit dem zu bestimmenden Analyten eingehen. Ein Beispiel für eine reaktive Festphase ist eine Streptavidin-beschichtete Festphase, an die ein biotinylierter Rezeptor binden kann. Der markierte Rezeptor kann direkt oder indirekt markiert sein. Der Analyt-spezifische Rezeptor in bindefähiger Form kann auch aus mehreren Komponenten bestehen, die nicht kovalent, sondern durch Affinitätswechselwirkung (Antikörper-Antigen; Streptavidin/Avidin-Biotin) miteinander verknüpft sind.

In einem Sandwich-Assay verwendet man als Entstörungsreagenz vorzugsweise einen unspezifischen Metallkomplex, der kein Elektrochemilumineszenzsignal liefert, z. B. einen Rhodium- oder einen Eisenkomplex.

Andererseits kann die Bestimmung des Analyten auch in einem kompetitiven Assay nach dem "labelled analog" oder "labelled receptor"-Format durchgeführt werden, z. B. in einem heterogenen kompetitiven Assay, wobei man
(a) die den Analyten enthaltende Probeflüssigkeit in Gegenwart einer reaktiven Festphase mit (a1) einem Analyt-spezifischen Rezeptor, der an die Festphase gebunden oder in einer an die Festphase gebunden oder in einer an die Festphase bindefähigen Form vorliegt, und einem Analytenanalog, das die Metallkomplex-Markierungsgruppe trägt, oder mit (a2) mit einem Analytenanalog, das an die Festphase gebunden oder in einer an die Festphase bindefähigen Form vorliegt, und einem Analytspezifischen Rezeptor, derdieMetallkomplex-Markierungsgruppe trägt, inkubiert,
(b) die Festphase gegebenenfalls von der Inkubationsflüssigkeit abtrennt und
(c) das Vorhandensein oder/und die Menge des Analyten in der Festphase oder/und in der flüssigen Phase durch Bestimmung der Markierung nachweist.

Als Analytenanalog wird eine Komponente verwendet, die mit dem zu bestimmenden Analyten um einen Analyt-spezifischen Rezeptor konkurriert.

Weiterhin betrifft die vorliegende Erfindung einen Reagenzienkit zur Durchführung des erfindungsgemäßen Verfahrens, umfassend einen lumineszenzfähigen Metallkomplex, gekoppelt an eine Analyt-spezifische Komponente als Markierungsgruppe und weiterhin als Entstörungsreagenz einen unspezifischen Metallkomplex, der eine mit der Markierungsgruppe chemisch verwandte Struktur besitzt. Der unspezifische Metallkomplex kann als Komplex alleine, als Komplex mit Linker oder/und als trägergebundener Komplex, wie oben erläutert, vorliegen.

Weiterhin wird die Erfindung durch die nachfolgenden Beispiele und Abbildungen erläutert. Es zeigen:
- Abb. 1: ein Reaktionsschema zur Herstellung von als Entstörungsreagenzien geeigneten Metallkomplexen mit oder ohne Linker,
- Abb. 2: ein Reaktionsschema zur Herstellung von als Entstörungsreagenzien geeigneten trägergebundenen Metallkomplexen,
- Abb. 3: ein Peptid-Ruthenium (bipyridyl)₃-Konjugat,
- Abb. 4: ein Testosteron-Ru(bipyridyl)₃-Konjugat,
- Abb. 5: ein Testosteron-Ru-bipyridyl-Konjugat mit Phenanthrolin als Komplexliganden,
- Abb. 6: einen Ru(bipyridyl)₃-Komplex mit Linker,
- Abb. 7: einen Ru(bipyridyl)₃-Komplex mit hydrophilem Linker,
- Abb. 8: einen Ru-bipyridyl-Komplex mit Phenanthrolin als Komplexliganden und einem hydrophilen Linker und
- Abb. 9: ein Testosteron-Peptid-Ru-(bipyridyl)₃-Konjugat.

### Beispiel 1

### Herstellung von Entstörungsreagenzien

### 1.1 Fe(II)bpy₃(PF₆)₂

FeCl₂ (64 mg) und 2,2'-Bipyridin (234 mg) werden in 5 ml H₂O/Ethanol (1:1) gelöst und bei Raumtemperatur geführt. Dann wird Ethanol abdestilliert, die wäßrige Lösung mit 2 Äquivalenten NH₄PF₆ versetzt. Der entstehende Niederschlag wird abgesaugt, nachgewaschen und getrocknet.
Ausbeute: 250 mg roter Feststoff

### 1.2 Ni(II)bpy₃(PF₆)₂

NiCl₂ (65 mg) wird in 3 ml H₂O gelöst und mit der Lösung von 2,2'-Bipyridin (240 mg) in 3 ml Ethanol versetzt. Nach 10 min werden die Lösungsmittel abdestilliert, der Rückstand mit Essigsäureethylester extrahiert, dann in 10 ml H₂O gelöst und mit NH₄PF₆ (200 mg) versetzt. Der Niederschlag wird abgesaugt, nachgewaschen und getrocknet.
Ausbeute: 380 mg rosa Feststoff
MS: M+: 671.1 (einfach geladenes Komplex⁺⁺ PF₆⁻-Kation)

### 1.3 Rh(III)bpy₂Cl₃

RhCl₃ (1,0 g) und 2,2-Bipyridin (1,5 g) werden mit 50 ml DMF versetzt und 3 Stunden gekocht. Es wird auf Raumtemperatur abgekühlt, der aufgefallene Feststoff abfiltriert, gewaschen und getrocknet.
Ausbeute: 1,41 g gelber Feststoff
MS: M+: 485 (einfach geladenes Komplex-Kation)

### 1.4 Rh(bpy₃(PF₆)₃

Rh(III)bpy₃Cl₂ (200 mg) und 2,2'-Bipyridin (60 mg) werden mit 20 ml H₂O versetzt und zum Sieden erhitzt. 20 µl Hydrazin-Hydrat-Lösung werden zugegeben und eine Stunde gekocht. Die Lösung wird eingeengt und mit NH₄PF₆ (220 mg) versetzt. Der Niederschlag wird abgesaugt, nachgewaschen und getrocknet.
Ausbeute: 310 mg gelber Feststoff

### 1.5 Fe(II)bpy₂-4-[4(4'-methyl-2,2'bipyridyl)]-bpybutansäure-N-Hydroxysuccinimidester (PF₆)₃

### a. Fe(II)bpy2-4-[4(4'-methyl-2,2'bipyridyl)]-butansäure (PF₆)₃

FeCl₂ (127 mg), 2,2'-Bipyridin (312 mg) und 4-[4(4'-Methyl-2,2'bipyrydin)]-butansäure (255 mg) werden in 7 ml H₂O/Ethanol (1:6) gelöst und bei Raumtemperatur gerührt. Dann werden die Lösungsmittel im Vakuum abdestilliert und der Rückstand mittels präparativer HPLC aufgereinigt (Polygosil PR 18,5 µ, Eluenten: A:H₂O+0,1% TFA, B:Acetonitril+0,1%TFA, Gradient: von 10% B langsam auf 100% B). Die sauberen Fraktionen werden lyophilisiert, das Lyophilisat in wenig Wasser gelöst, mit wäßriger NH₄PF₆-Lsg. versetzt, der Niederschlag abgesaugt, gewaschen und getrocknet.
Ausbeute: 140 mg roter Feststoff

### b. Fe(II)bpy₂-4-[4(4'-methyl-2,2'bipyridyl)]-bpybutansäure-N-Hydroxysuccinimidester (PF₆)₃

Fe(II)-bpy2-bpybutansäure (PF₆)₂(53 mg) und N-Hydroxysuccinimid (8,7 mg) werden in 5 ml CH₂Cl₂ gelöst, 2-(4-Morpholinyl)-ethylisocyanid (8,3 µl) wird zugetropft und die Lösung bei Raumtemperatur gerührt. Nach vollständiger Umsetzung wird das Lösungsmittel im Vakuum abdestilliert, der Rückstand in Aceton gelöst, das Produkt durch Zugabe von THF/Diethylether ausgefällt, abfiltriert und getrocknet.
Ausbeute: 52 mg roter Feststoff
MS: M⁺: 866.1 (einfach geladenes Komplex⁺⁺-PF₆⁻-Kation)
¹H-NMR: O.K.

### 1.6 Rh(III)bpy₂-4-[4(4'-methyl-2,2'bipyridyl)]-butansäure (PF₆)₃

Rh(bpy)₂Cl₃ (6,3 g) und 4-[4(4'-Methyl-2,2'bipyridyl)]-butansäure (3,5 g) werden in 400 ml einer Wasser/Ethanol-Mischung (9:1) bis zum Sieden erhitzt. Hydrazin-Hydrat-Lsg. (0,73 ml, 80%ig) wird zugetropft und dann 3,5 Stunden unter Rückfluß gekocht. Die Lösung wird abgekühlt, auf ca. 50 ml eingeengt und über eine SP Sephadex C-25-Säule aufgereinigt. (Eluenten: zuerst HCl (c: 0,1 mol/l), dann HCl (c: 0,1 mol/l)/NaCl (c: 0,5 mol/l)). Die sauberen Fraktionen werden eingeengt und mit einer wäßrigen Ammoniumhexafluorphosphat-Lösung versetzt. Der Niederschlag wird abgesaugt, gewaschen und getrocknet.
Ausbeute: 11,6 g weißer Feststoff,
MS: M+: 961.1 (einfach geladenes Komplex ⁺⁺⁺ 2(PF₆)⁻-Kation)
¹H-NMR: O.K.

### 1.7 Rh(III)bpy₂-4-[4(4'-methyl-2,2'bipyridyl)]-bpybutansäure-N-Hydroxysuccinimidester (PF₆)₃

Rh(III)bpy₂-bpybutansäure (PF₆)₃ (10g) und N-Hydroxysuccinimid (1,05 g) werden in 100 ml Acetonitril gelöst. Dann wird 2-(4-Morpholinyl)-ethylisocyanid (1,4 ml) zugetropft. Nach vollständiger Umsetzung wird die Lösung eingeengt, mit Chloroform versetzt und intensiv gerührt. Dann wird der Überstand vom zähen Feststoff abgegossen, dieser erneut in wenig Acetonitril gelöst und der Vorgang wiederholt. Lösungsmittelreste werden im Hochvakuum entfernt.
Ausbeute: 11 g beiger Feststoff
MS: M+: 1058.0 (einfach geladenes Komplex ⁺⁺⁺(PF₆)₂⁻-Kation)
¹H-NMR: o.k.

### 1.8 Ru(II)bpyCO-ε-Lys-Fmoc

Fmoc-Lysin-OH*HCl (3,8 g) und Triethylamin (2,6 ml) in 70 ml DMF werden unter Rühren zu Ru (bpy)₂-bpyCO-NHS-Ester (siehe EP-A-580 979) (9,9 g) in 50 ml DMF getropft. Nach 1 Stunde wird das DMF im Hochvakuum abdestilliert, der Rückstand in Aceton gelöst, die Lösung filtriert, das Filtrat auf ca. 50 ml eingeengt und unter starkem Rühren mit Diethylether versetzt. Nach 14 h wird der Überstand vom ausgefallenen Feststoff abgegossen, dieser nochmals mit Diethylether extrahiert, Lösungsmittelreste entfernt. Ausbeute: 14 g roter Feststoff

Die Verbindung kann direkt in die Peptidsynthese eingebracht werden (vgl. auch DE-44 39 345.8, wo die Synthese von definiert eingebauten Markierungsgruppen und Haptenen beschrieben ist). Ausgehend vom NHS-Ester können auch direkt Hapten-Derivate etc. hergestellt werden. Andererseits kann die Verbindung gegebenenfalls nach Abspaltung der Fmoc-Schutzgruppe direkt als Entstörungsreagenz eingesetzt werden.

### 1.9 Metallkomplex-Peptid-Konjugat

Die Peptid-Basis (20 mg) wird in DMF gelöst und mit 30 µl Triethylamin versetzt. Dann werden unter Rühren äquimolare Mengen des entsprechenden Metallkomplex-NHS-Esters zugegeben und weiter 1 h bei Raumtemperatur gerührt. Danach wird das Lösungsmittel im Vakuum abdestilliert und der Rückstand mittels präparativer HPLC aufgereinigt (Polygosil RP 18, 5µ, Eluenten: A: H₂O+0,1% TFA, B: Acetonitril+0,1%TFA, Gradient: von 10% B langsam auf 100% B).

Mit dem oben beschriebenen Verfahren wurde beispielsweise folgendes Metallkomplex-Peptid-Konjugat hergestellt (K = Lys, E = Glu, U = β-Ala):
Peptid-Sequenz:Ac-KUEUEUEUEUEUK-NH₂
NHS-Ester: Ru-(bpy)₂-bpyCO-NHS-Ester
Ausbeute: 60 mg Ac-K-Ru(bpy)₃-UEUEUEUEUEU-K-Ru(bpy)₃-NH₂
MS: M+:1344.5 -- doppelt geladenes Komplex-Kation (2xRu⁺⁺COO⁻)
¹H-NMR: O.K.

### Beispiel 2

### Herstellung von Metallkomplex-Konjugaten mit IgG und Antikörperfragmenten

Von Rinder-IgG und Schaf-IgG können übliche marktgängige Präparate verwendet werden.

F(ab')₂- und Fab-Antikörperfragmente wurden nach Johnstone and Thrope (immunochemistry in Practice S. 61f, Blackwell Scientific, 1987) durch Spaltung mit Pepsin bzw. Papain aus dem entsprechenden IgG hergestellt und gereinigt.

Polymerisierte Rinder-Fab-Fragmente wurden durch Vernetzung von Rinder-Fab mit Disuccinimidylsuberat nach EP-A-0 269 092 hergestellt.

Die Umsetzung von aktiven Estern der in Beispiel 1 angegebenen Metall-Komplexe mit den verschiedenen IgG bzw. Antikörperfragment-Präparaten erfolgte nach dem Schema, das im folgenden für 3 Beispiele beschrieben ist:

### 2.1 Ru(bpy)₃-Konjugat mit monoklonalem Anti-TSH-F(ab')₂-Fragment Stöchometrie 7.5:1)

5 mg monoklonales Anti-TSH-F(ab')₂-Fragment werden in 1 ml 0,15 M K-Phosphat-Puffer, 0,15 M NaCl, pH 7,8, gelöst. Unmittelbar vor Gebrauch löst man 5 mg Ru(bpy)₂-bpy-CO-NHS-Ester (Fa. IGEN Inc., Rockville, USA) in 0,75 ml wasserfreiem Dimethylsulfoxid. Um ein Molverhältnis von 7,5:1 zu erhalten, bezogen auf die Molekulargewichte 1075 für Ru(bpy)₂-bpy-CO-NHS-Ester und 100.000 für F(ab')₂, werden 0,369 mg Ru(bpy)₂-bpy-CO-NHS-Ester (59,4 µl) zur F(ab')₂-Lösung unter Rühren zupipettiert. Das Reaktionsgefäß wird 60 min. bei 25 °C inkubiert. Zum Stop der Umsetzung pipettiert man 10 µl einer 1 M Lysin-Lösung hinzu. Der Ansatz wird 24 Std. gegen 25 mM K-Phosphatpuffer/0,1 M NaCl, pH 7,0, dialysiert, dann lyophilisiert.
Ausbeute: 4,4 mg Anti-TSH-F(ab')₂-Ru(bpy)₃²⁺
Über Extinktionsmessung bei 455 nm (ε=13.7) ergibt sich ein Molverhältnis an eingebauter Markierung von 5,8-6,8:1 [Ru:F(ab')₂].

### 2.2 Rinder-IgG-Rh(bpy)₃ 1:10

50 mg Rinder IgG werden in 5 ml 0,15 M K-Phosphat-Puffer, 0,15 M NaCl, pH 7,8, gelöst. Unmittelbar vor Gebrauch löst man 8 mg Rh(bpy)₂-bpy-CO-NHS-Ester in 0,5 ml wasserfreiem Dimethylsulfoxid. Um ein Molverhältnis von 10:1 zu erhalten, bezogen auf die Molekulargewichte 1203 für Rh(bpy)₂-bpy-CO-NHS-Ester und 150 000 für IgG, werden 4 mg Rh(bpy)₂-bpy-CO-NHS-Ester (250 µl) zur IgG-Lösung unter Rühren zupipettiert. Das Reaktionsgefäß wird 60 min. bei 25 °C inkubiert. Zum Stop der Umsetzung pipettiert man 10 µl einer 1 M Lysin-Lösung hinzu. Der Ansatz wird 24 Std. gegen 25 mM K-Phosphatpuffer/0,1 M NaCl, pH 7,0, dialysiert, dann lyophilisiert.
Ausbeute: 46 mg Rinder-IgG-Rh(bpy)₃³⁺.

### 2.3 Rinder-Fab-Rh(bpy)₃-Polymer 1:3.5

50 mg Rinder-Fab-Polymer werden in 5 ml 0,15 M K-Phosphat-Puffer, 0,15 M NaCl, pH 7,8, gelöst. Unmittelbar vor Gebrauch löst man 8 mg (Rh(bpy)₂-bpy-CO-NHS-Ester in 0,5 ml wasserfreiem Dimethylsulfoxid. Um ein Molverhältnis von 3,5:1 zu erhalten, bezogen auf die Molekulargewichte 1203 für Rh(bpy)₂-bpy-CO-NHS-Ester und 50.000 für Fab (als Einzelbaustein im Polymer), werden 4,2 mg Rh(bpy)₂-bpy-CO-NHS-Ester (263 µl) zur Fab-Polymer-Lösung unter Rühren zupipettiert. Das Reaktionsgefäß wird 60 min. bei 25 °C inkubiert. Zum Stop der Umsetzung pipettiert man 10 µl einer 1M Lysin-Lösung hinzu. Der Ansatz wird 24 Std. gegen 25 mM K-Phosphatpuffer/0,1 M NaCl, pH 7,0, dialysiert, dann lyophilisiert.
Ausbeute: 38 mg Rinder-Fab-Rh(bpy)₃Polymer

### Beispiel 3

### TBK-Test

### Testsystem:

Im TBK-Test wird die Thyroxin-Bindekapazität von Serum bestimmt.

Im ersten Reaktionsschritt wird eine Serumprobe mit einem Reagenz versetzt, das T4 enthält. Das Reagenz enthält als wesentliche Komponenten außer T4 ein T4-Polyhapten-Biotin (T4-Bio-Ph; 70 ng/ml). Das zugesetzte T4 bindet in einer Menge abhängig von der Bindekapazität der Probe an deren Bindeproteine.

In einem zweiten Reaktionsschritt wird ein Puffer mit einem polyklonalen Anti-T4-Antikörper-Ru-bipyridyl-Derivat (100 ng/ml) zugesetzt. Außerdem werden mit Streptavidin beschichtete Mikro-partikel zugegeben. Das aus der ersten Reaktion überschüssige T4 konkurriert mit dem T4-Bio-Ph um den markierten Antikörper. Der an das T4 Polyhapten gebundene markierte Antikörper wird über das Biotin an die streptavidinbeschichteten Mikropartikel gebunden.

In einer Meßzelle werden die Mikropartikel von der flüssigen Phase getrennt. Der in analytabhängiger Menge an die Mikropartikel gebundene markierte Antikörper bleibt hierbei an der Festphase gebunden. In der Meßzelle wird das ECL-Signal proportional der Konzentration des Ru-bipyridyl-Komplexes elektrochemisch erzeugt und über eine Kalibrationskurve die Analytkonzentration ermittelt.

### Untersuchung auf Entstörwirkung:

In parallel durchgeführten Experimenten wurden Proben (bekannte Störproben und normale Proben) ohne bzw. mit Entstörsubstanz vermessen. Als Referenz wurden die Proben zusätzlich in einer unabhängigen Methode vermessen. Die eingesetzten Derivate und Konzentrationen sind in der Tabelle 1 aufgeführt.

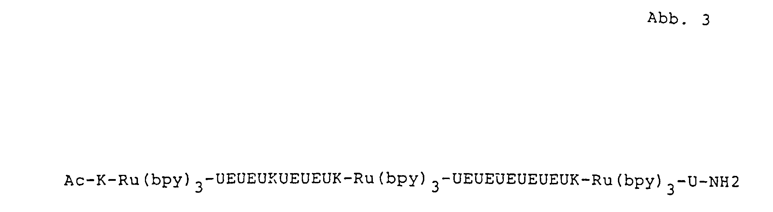

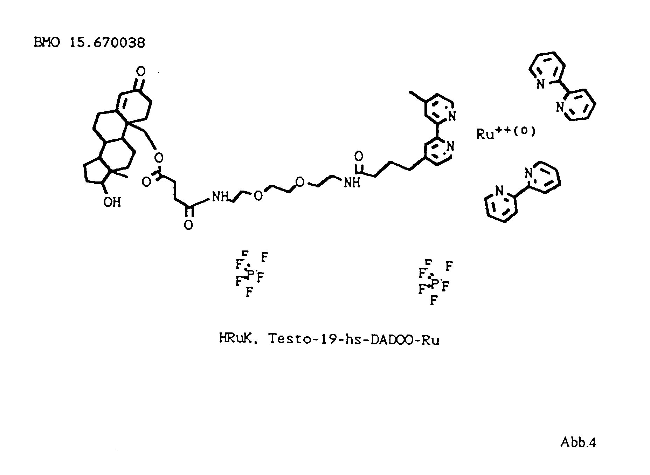

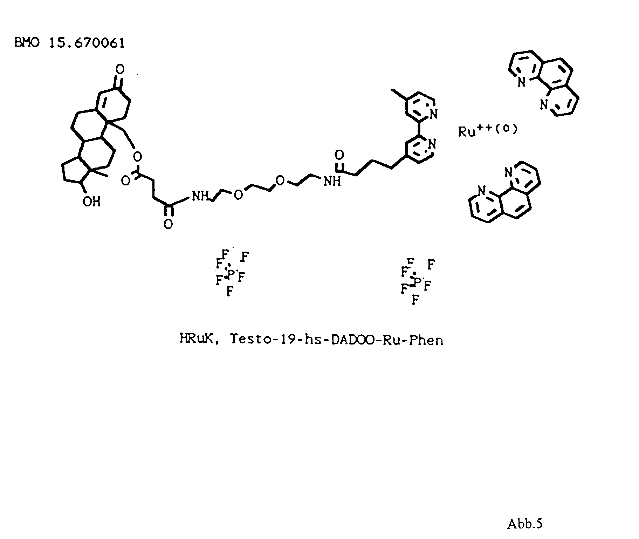

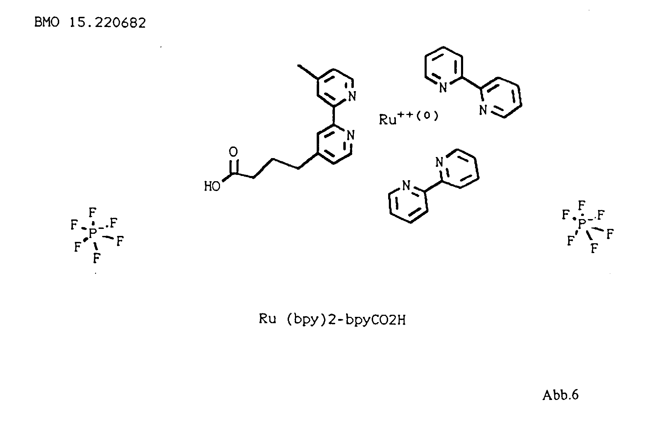

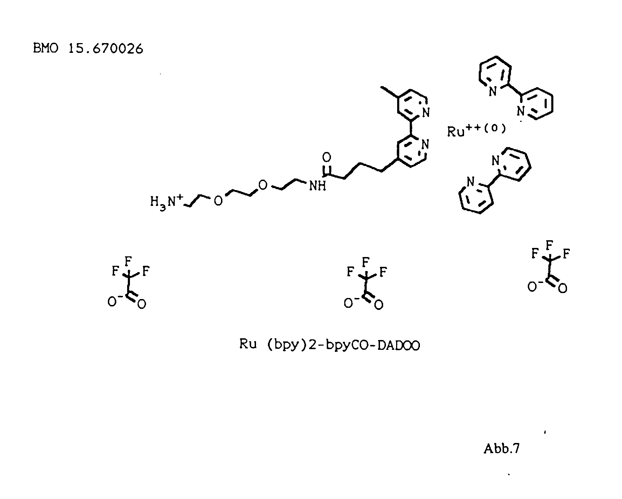

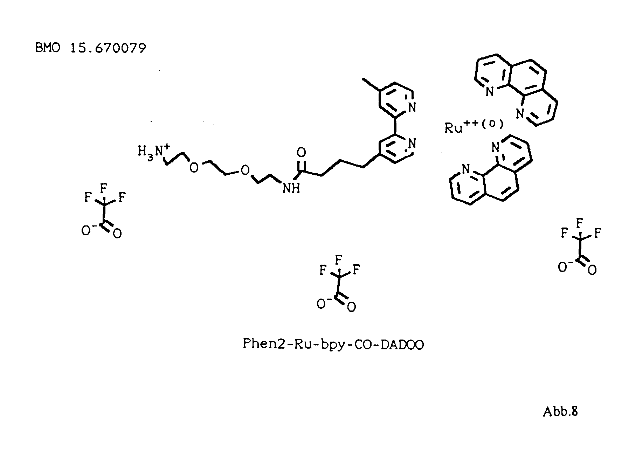

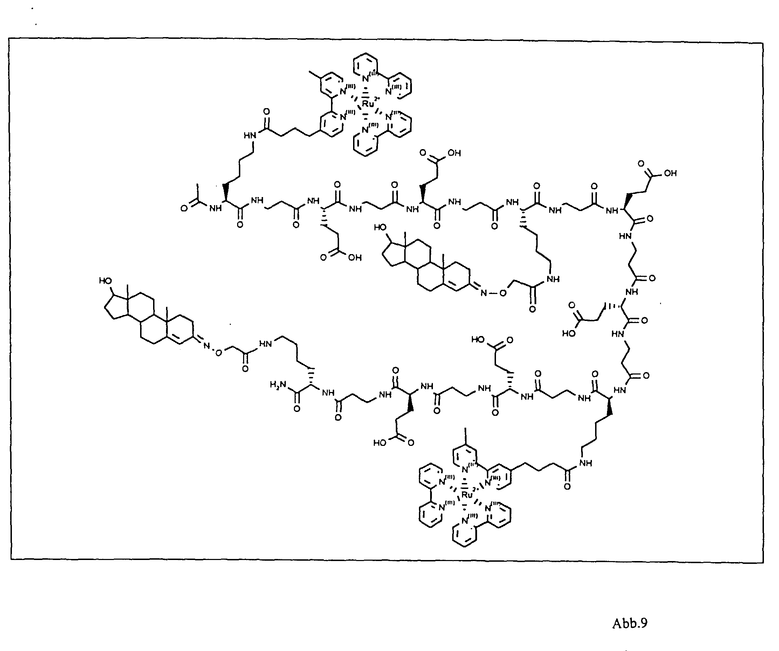

Es wurden folgende Entstörungsreagenzien verwendet:
1: unspezifischer monoklonaler Antikörper (IgG)-Os(bpy)₃-Derivat (Stöchiometrie von Träger zur Markierung 1 : 12,5)
2: Peptid-Ru(bpy)₃-Konjugat (vgl. Abb. 3)
3: unspezifischer polyklonaler Rinderantikörper-(Fab-Fragment)-Os(bpy)₃-Derivat (Stöchiometrie 1 : 2,5)
4: vernetzter unspezifischer polyklonaler Rinderantikörper-(Fab-Fragment)-Os(bpy)₃-Derivat (Stöchiometrie 1 : 2,5)
5: vernetzter unspezifischer polyklonaler Rinderantikörper (Fab-Fragment)-Fe(bpy)₃-Derivat (Stöchiometrie 1 : 2,5)
6: Lysin-Ru(bpy)₃ (vgl. Beispiel 1.8)
7: unspezifischer polyklonaler Rinderantikörper (IgG) Rh-(bpy)₃-Derivat (Stöchiometrie (1 : 6)
8: unspezifischer polyklonaler Rinderantikörper (IgG)-Rh(bpy)₃-Derivat (Stöchiometrie 1 : 10)
9: vernetzter unspezifischer polyklonaler Rinderantikörper (Fab-Fragment)-Rh(bpy)₃-Derivat (Stöchiometrie 1 : 2)
10: vernetzter unspezifischer polyklonaler Rinderantikörper (Fab-Fragment)-Rh(bpy)₃-Derivat (Stöchiometrie 1 : 3,5)
11: unspezifischer polyklonaler Schafantikörper (IgG)-Rh(bpy)₃-Derivat (Stöchiometrie 1 : 6)
12: unspezifischer polyklonaler Schafantikörper (IgG) Rh(bpy)₃-Derivat (Stöchiometrie 1 : 10)
13: Testosteron-19-hs-Diaminodioxaoctan-Ru(bpy)₃-Konjugat (bpy)₃-Konjugat (vgl. Abb. 4)
14: Peptid-Ru(bpy)3-Konjugat (vgl. Beispiel 1.9)
15: Testosteron-19-hs-DADOO-Ru-Bipyridin-(Phenanthrolin)₂-Konjugat (vgl. Abb. 5)
16: Ru(bpy)₂-bpy-CO₂H (vgl. Abb. 6)
17: Ru(bpy)2-bpy-CO-DADOO (vgl. Abb. 7)
18: Ru(Phenanthrolin)2-bpy-CO-DADOO (vgl. Abb. 8)
19: Testosteron-Peptid-Ru(bpy)₃-Konjugat (vgl. Abb. 9)

### Ergebnis:

In Abhängigkeit von der Art und Konzentration der Entstörungskomponente werden Störseren gegenüber den unentstörten Reagenzien besser wiedergefunden. Der Zusatz von Entstörungsreagenzien bewirkt keine signifikante Verschlechterung der Testergebnisse bei Normalseren.

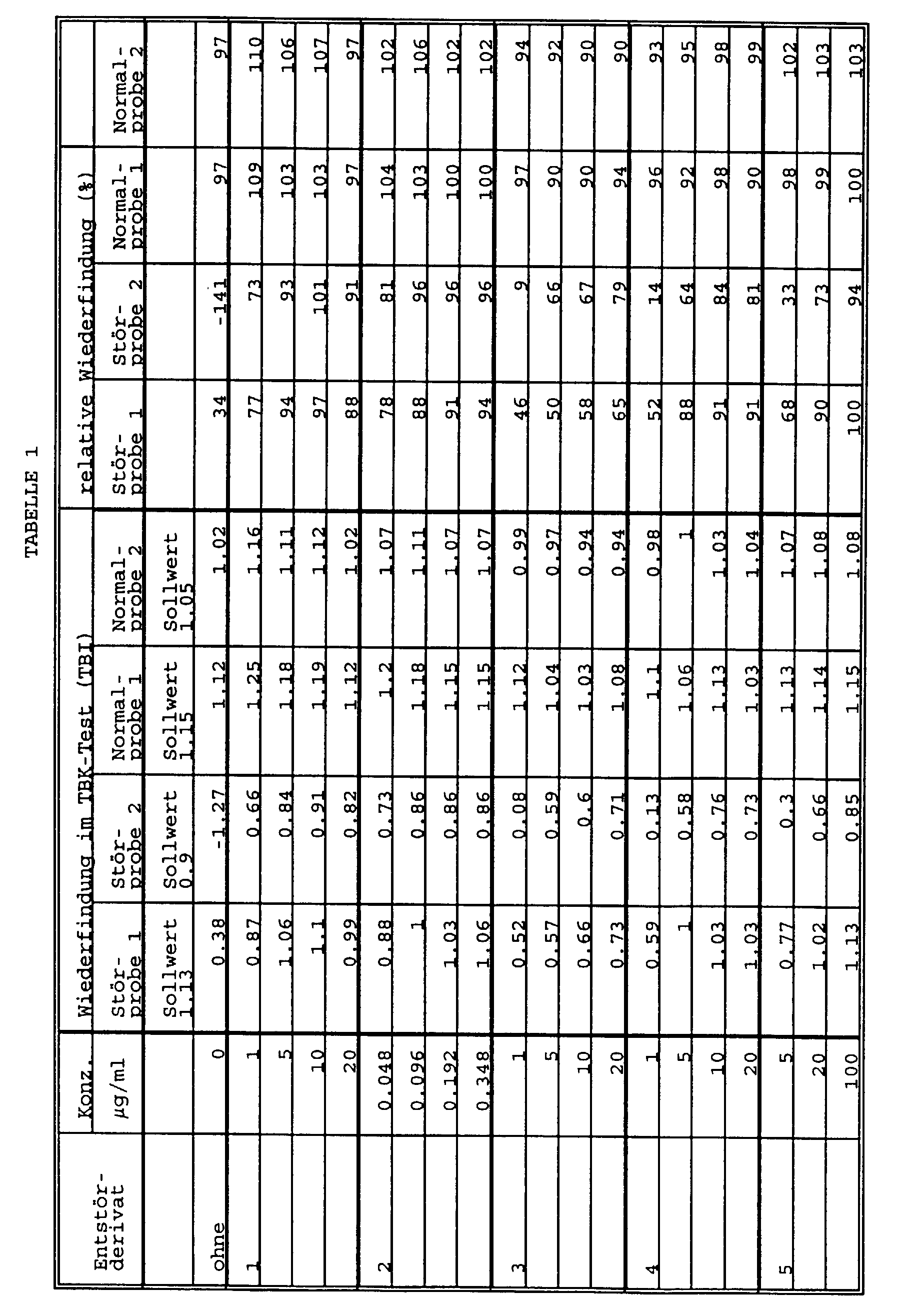

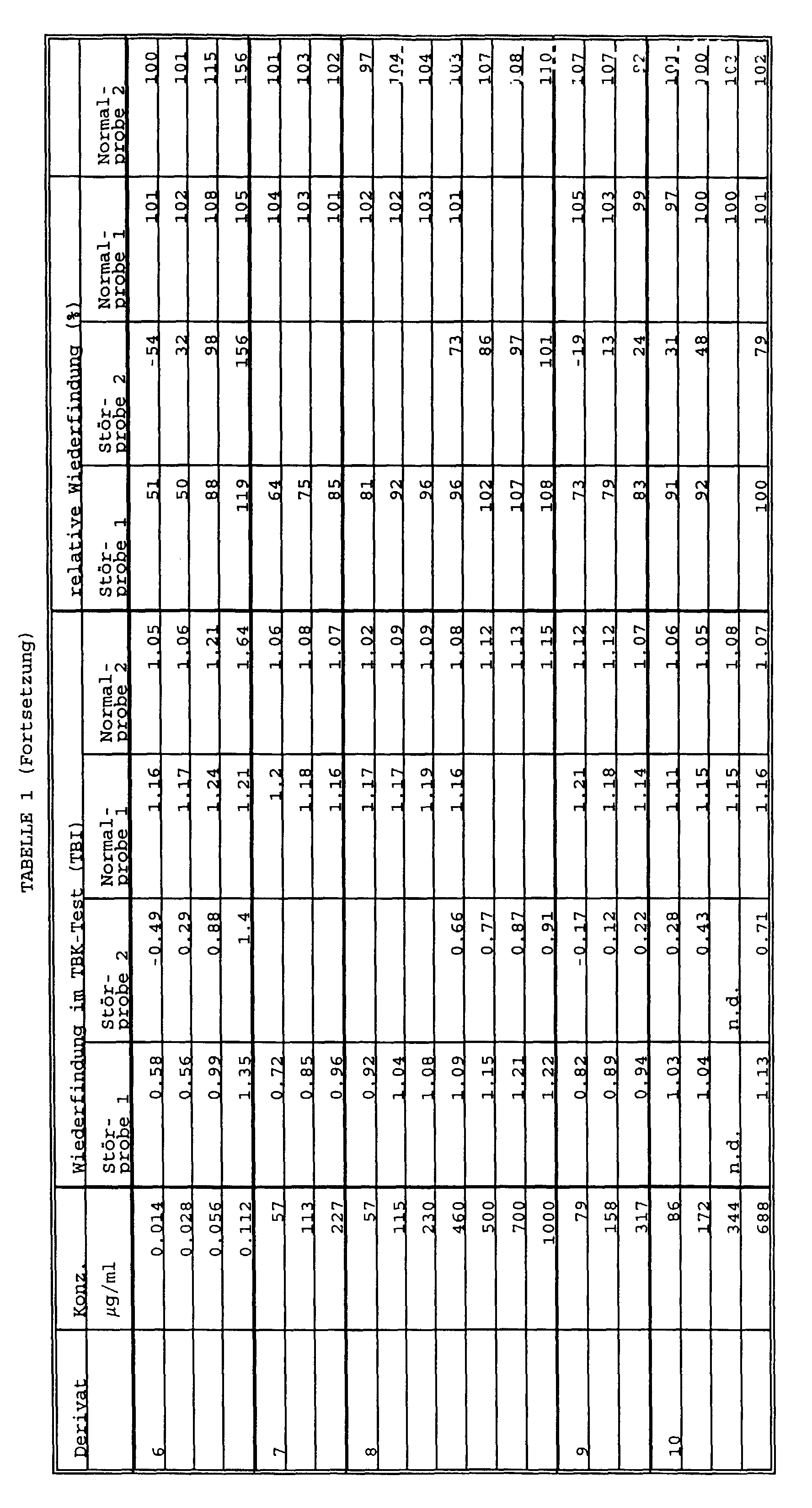

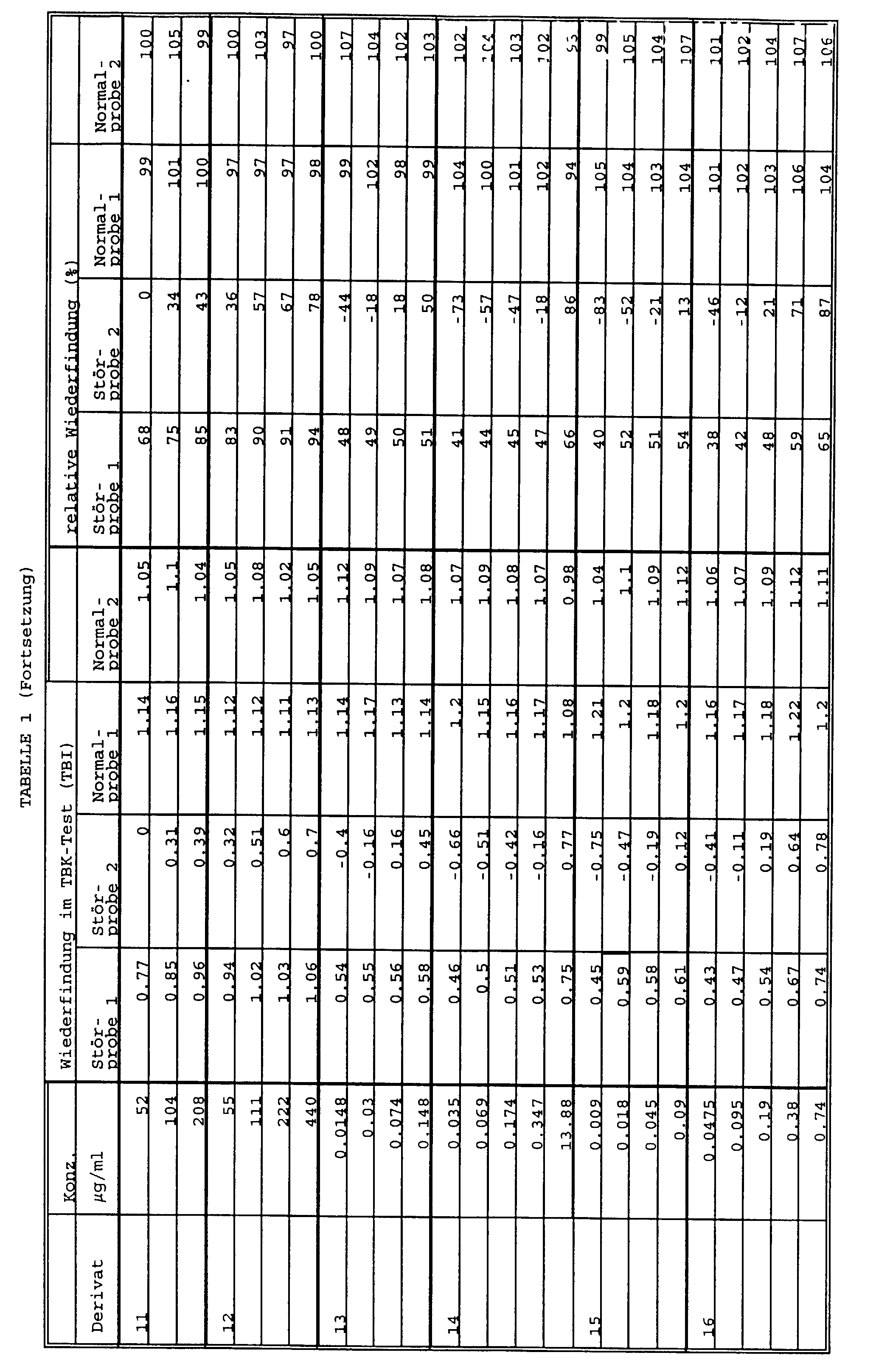

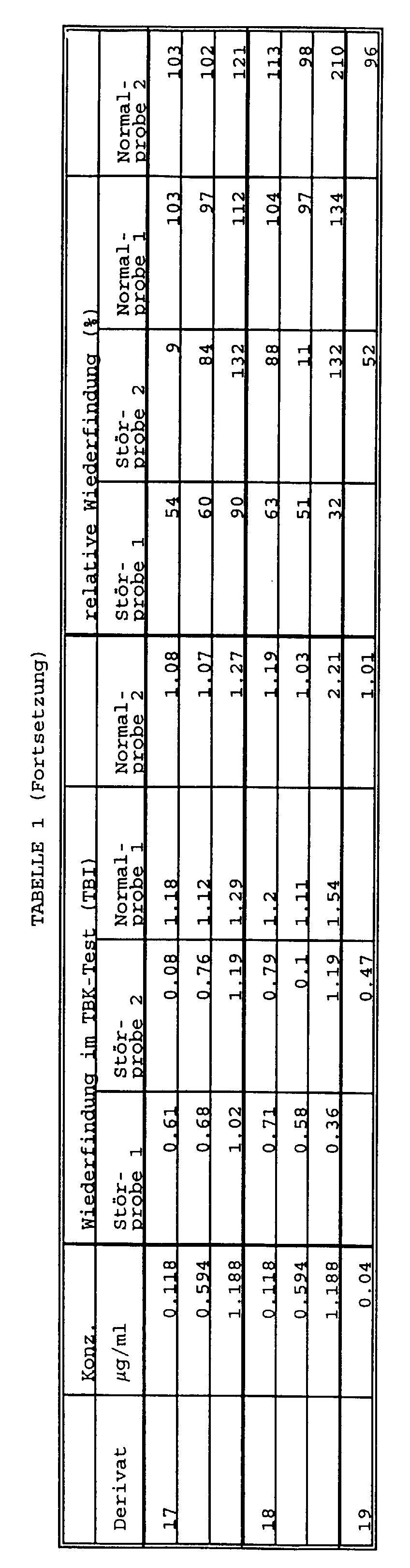

### Beispiel 4

### FT4-Test

### Testdurchführung:

Im FT4-Test wird der freie Anteil von Thyroxin im Serum bestimmt.

Im ersten Schritt wird eine Probe in einem Inkubationsgefäß mit einem polyklonalen Anti-T4-Antikörper-Fab-Fragment-Ru(bpy)₃-Konjugat (50 ng/ml) in einem Phosphatpuffer inkubiert. Dabei reagiert das in der Probe vorhandene freie Thyroxin mit dem Antikörperderivat.

Im zweiten Reaktionsschritt wird eine Komponente, die über eine Linkerstruktur Biotin an T4 gebunden enthält (2,5 ng/ml in Phosphatpuffer) zugesetzt. Außerdem werden mit Streptavidin beschichtete Mikropartikel zugegeben.

Der Anteil des Antikörperderivats, der kein freies T4 aus der Probe gebunden hat, kann mit dem T4-Biotinderivat reagieren und über Biotin-Streptavidin-Wechselwirkung an die Mikropartikel gebunden werden. Nach Abtrennung der Mikropartikel wird auf einer Elektrode das ECL-Signal proportional der gebundenen Menge an markiertem Antikörperderivat erzeugt. Das so generierte und gemessene Signal wird an einer Kalibrationskurve abgelesen und somit die Analytkonzentration ermittelt.

In Tabelle 2 sind Ergebnisse dargestellt, bei denen eine Probe (bekannte Störproben und normale Proben) mit bzw. ohne Entstörungsreagenz vermessen wurde. Als Referenz wurden die Proben in einem Immunoassay, der eine richtige klinische Klassifizierung der Proben ergibt, vermessen.

Es wurden die in Beispiel 2 beschriebenen Entstörungsreagenzien 1, 2, 4, 5, 6, 8, 14, 16 und 19 verwendet. Weiterhin wurde als Entstörungsreagenz folgender Metallkomplex verwendet:
20: vernetzter unspezifischer polyklonaler KaninchenantikörperFab-Fragment) Ru(bpy)₃-Derivat (Stöchiometrie 1 : 2,5).

### Ergebnis:

Durch Zusatz von geeigneten Entstörderivaten werden die im nicht entstörten Reagenz falsch wiedergefundenen Serumproben besser wiedergefunden. Eine signifikante Störung bei der Bestimmung von Normalseren wird im allgemeinen nicht gefunden.

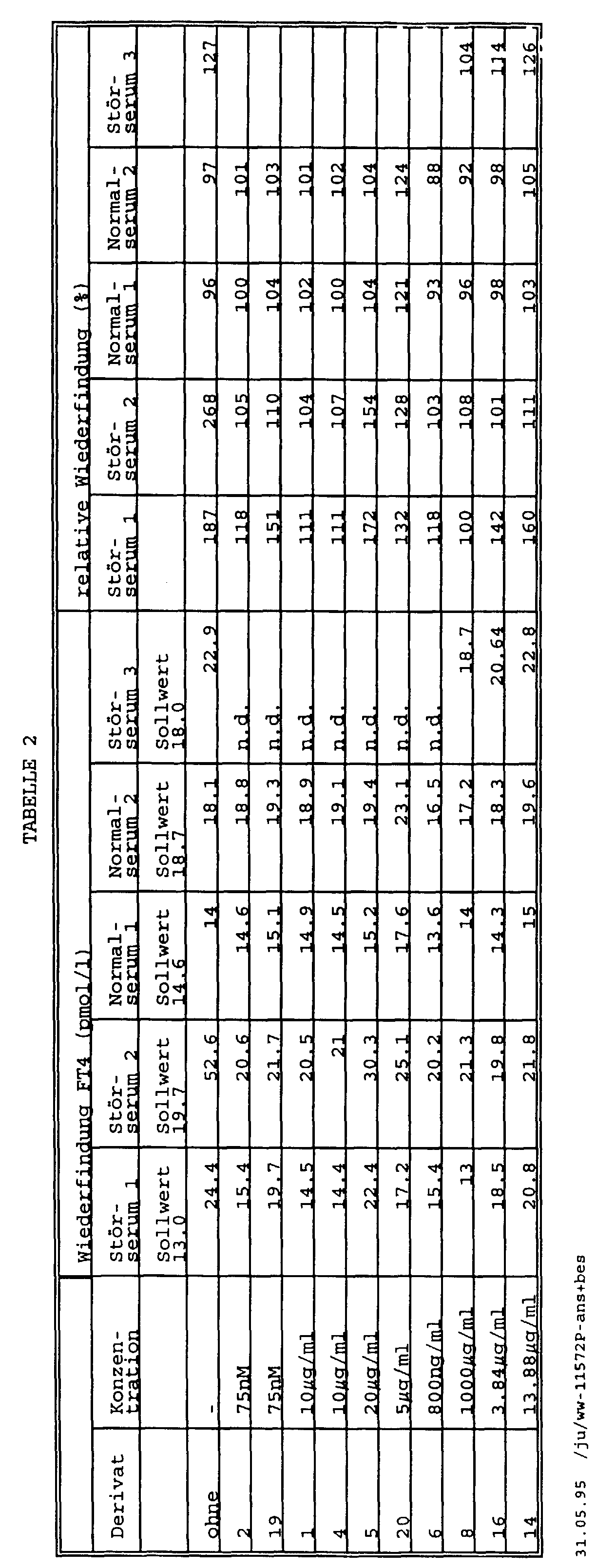

## Patentansprüche

1. Verfahren zur Bestimmung eines Analyten in einer Probeflüssigkeit mit einem lumineszenzfähigen Metallkomplex als Analyt-spezifischer Markierungsgruppe zur Erzeugung eines Meßsignals,
**dadurch gekennzeichnet,**
daß man weiterhin einen unspezifischen Metallkomplex als Entstörungsreagenz zusetzt, der eine mit der Markierungsgruppe chemisch verwandte Struktur besitzt.

2. Verfahren nach Anspruch 1,
**dadurch gekennzeichnet,**
daß die Analyt-spezifische Markierungsgruppe ausgewählt wird aus Übergangsmetall- und Seltenerdmetallkomplexen.

3. Verfahren nach Anspruch 1 oder 2,
**dadurch gekennzeichnet,**
daß der unspezifische Metallkomplex ausgewählt wird aus Übergangsmetall- und Seltenerdmetallkomplexen.

4. Verfahren nach einem der Ansprüche 1 bis 3,
**dadurch gekennzeichnet,**
daß die Liganden in den Metallkomplexen ausgewählt werden aus aromatischen heterocyclischen Polydentatliganden.

5. Verfahren nach einem der Ansprüche 1 bis 4,
**dadurch gekennzeichnet,**
daß der unspezifische Metallkomplex als Komplex alleine, als Komplex mit Linker oder/und als trägergebundener Komplex zugesetzt wird.

6. Verfahren nach Anspruch 5,
**dadurch gekennzeichnet,**
daß man als Träger eine nicht mit der Bestimmung des Analyten interferierende Substanz verwendet.

7. Verfahren nach Anspruch 6,
**dadurch gekennzeichnet,**
daß man einen trägergebundenen Komplex verwendet, der eine Stöchiometrie von Komplex zu Träger von größer oder gleich 1 : 1 aufweist.

8. Verfahren nach einem der Ansprüche 1 bis 7,
**dadurch gekennzeichnet,**
daß man den unspezifischen Metallkomplex in einem stöchiometrischen Überschuß bezüglich der Analyt-spezifischen Markierungsgruppe einsetzt.

9. Verfahren nach Anspruch 1,
**dadurch gekennzeichnet,**
daß die Bestimmung des Analyten in einem heterogenen Sandwich-Assay durchgeführt wird, wobei man
(a) die den Analyten enthaltende Probeflüssigkeit in Gegenwart einer reaktiven Festphase mit mindestens zwei Analyt-spezifischen Rezeptoren inkubiert, von denen einer an die Festphase gebunden oder in einer an die Festphase bindefähigen Form vorliegt und ein anderer die Metallkomplex-Markierungsgruppe trägt,
(b) die Festphase gegebenenfalls von der Inkubationsflüssigkeit abtrennt und
(c) das Vorhandensein oder/und die Menge des Analyten in der Festphase oder/und in der flüssigen Phase durch Bestimmung der Markierung nachweist.

10. Verfahren nach Anspruch 1,
**dadurch gekennzeichnet**,
daß die Bestimmung des Analyten in einem heterogenen kompetitiven Assay durchgeführt wird, wobei man
(a) die den Analyten enthaltende Probeflüssigkeit in Gegenwart einer reaktiven Festphase mit (a1) einem Analyt-spezifischen Rezeptor, der an die Festphase gebunden oder in einer an die Festphase gebunden oder in einer an die Festphase bindefähigen Form vorliegt, und einem Analytenanalog, das die Metallkomplex-Markierungsgruppe trägt, oder mit (a2) mit einem Analytenanalog, das an die Festphase gebunden oder in einer an die Festphase bindefähigen Form vorliegt, und einem Analyt-spezifischen Rezeptor, der die Metallkomplex-Markierungsgruppe trägt, inkubiert,
(b) die Festphase gegebenenfalls von der Inkubationsflüssigkeit abtrennt und
(c) das Vorhandensein oder/und die Menge des Analyten in der Festphase oder/und in der flüssigen Phase durch Bestimmung der Markierung nachweist.

11. Reagenzienkit zur Durchführung eines Verfahrens nach einem der Ansprüche 1 bis 10,
umfassend einen lumineszenzfähigen Metallkomplex, gekoppelt an eine Analyt-spezifische Komponente als Markierungsgruppe und weiterhin als Entstörungsreagenz einen unspezifischen Metallkomplex, der eine mit der Markierungsgruppe chemisch verwandte Struktur besitzt.

12. Reagenzienkit nach Anspruch 11,
**dadurch gekennzeichnet**,
daß der unspezifische Metallkomplex als Komplex alleine, als Komplex mit Linker oder/und als trägergebundener Komplex vorliegt.

## Claims

1. Method for the determination of an analyte in a sample liquid using a metal complex capable of luminescence as an analyte-specific marker group for the production of a measuring signal,
**wherein**
an unspecific metal complex is additionally added as an interference elimination reagent that has a structure which is chemically related to the marker group.

2. Method as claimed in claim 1,
**wherein**
the analyte-specific marker group is selected from transition metal and rare earth metal complexes.

3. Method as claimed in claim 1 or 2,
**wherein**
the unspecific metal complex is selected from transition metal and rare earth metal complexes.

4. Method as claimed in one of the claims 1 to 3,
**wherein**
the ligands in the metal complexes are selected from aromatic heterocyclic polydentate ligands.

5. Method as claimed in one of the claims 1 to 4,
**wherein**
the unspecific metal complex is added as a complex alone, as a complex with a linker or/and as a carrier-bound complex.

6. Method as claimed in claim 5,
**wherein**
a substance that does not interfere with the determination of the analyte is used as the carrier.

7. Method as claimed in claim 6,
**wherein**
a carrier-bound complex is used which exhibits a stoichiometry of complex to carrier of more than or equal to 1 : 1.

8. Method as claimed in one of the claims 1 to 7,
**wherein**
the unspecific metal complex is used in a stoichiometric excess relative to the analyte-specific marker group.

9. Method as claimed in claim 1,
**wherein**
the determination of the analyte is carried out in a heterogeneous sandwich assay in which
(a) the sample liquid containing the analyte is incubated in the presence of a reactive solid phase with at least two analyte-specific receptors one of which is bound to the solid phase or is present in a form capable of binding to the solid phase and another carries the metal complex marker group,
(b) the solid phase is optionally separated from the incubation liquid and
(c) the presence or/and the amount of the analyte in the solid phase or/and in the liquid phase is detected by determining the label.

10. Method as claimed in claim 1,
**wherein**
the determination of the analyte is carried out in a heterogeneous competitive assay in which
(a) the sample liquid containing the analyte is incubated in the presence of a reactive solid phase with (al) an analyte-specific receptor which is present bound to the solid phase or in a form bound to the solid phase or capable of binding to the solid phase and with an analyte analogue which carries the metal complex marker group, or with (a2) an analyte analogue which is bound to the solid phase or is present in a form capable of binding to the solid phase and with an analyte-specific receptor which carries the metal complex marker group
(b) the solid phase is optionally separated from the incubation liquid and
(c) the presence or/and the amount of the analyte in the solid phase or/and in the liquid phase is detected by determining the label.

11. Reagent kit for carrying out a method as claimed in one of the claims 1 to 10,
comprising a metal complex capable of luminescence which is coupled to an analyte-specific component as the marker group and additionally an unspecific metal complex as the interference elimination reagent which has a structure that is chemically related to the marker group.

12. Reagent kit as claimed in claim 11,
**wherein**
the unspecific metal complex is present as a complex alone, as a complex with a linker or/and as a carrier-bound complex.

## Revendications

1. Procédé de dosage d'un analyte dans un échantillon liquide à l'aide d'un complexe métallique luminescent en tant que groupe marqueur spécifique de l'analyte pour la production d'un signal de mesure, caractérisé en ce que l'on ajoute en outre un complexe métallique non spécifique comme réactif destiné à éliminer les réactions parasites, qui possède une structure chimiquement apparentée à celle du groupe marqueur.

2. Procédé selon la revendication 1, caractérisé en ce que le groupe marqueur spécifique de l'analyte est choisi parmi des complexes de métaux de transition et de métaux du groupe des terres rares.

3. Procédé selon la revendication 1 ou 2, caractérisé en ce que le complexe métallique non spécifique est choisi parmi des complexes de métaux de transition et de métaux du groupe des terres rares.

4. Procédé selon l'une des revendications 1 à 3, caractérisé en ce que les ligands des complexes métalliques sont choisis parmi des ligands hétérocycliques aromatiques à plusieurs liaisons de coordination.

5. Procédé selon l'une des revendications 1 à 4, caractérisé en ce que l'on ajoute le complexe métallique non spécifique sous forme du complexe seul, sous forme du complexe avec un segment de liaison et/ou sous forme du complexe lié à un support.

6. Procédé selon la revendication 5, caractérisé en ce que l'on utilise comme support une substance ne gênant pas le dosage de l'analyte.

7. Procédé selon la revendication 6, caractérisé en ce que l'on utilise un complexe lié à un support présentant une stoechiométrie du complexe au support supérieure ou égale à 1 : 1.

8. Procédé selon l'une des revendications 1 à 7, caractérisé en ce que l'on utilise le complexe métallique non spécifique en un excès stoechiométrique par rapport au groupe marqueur spécifique de l'analyte.

9. Procédé selon la revendication 1, caractérisé en ce que l'on effectue le dosage de l'analyte par une analyse en sandwich hétérogène, dans laquelle
(a) on met à incuber l'échantillon liquide contenant l'analyte en présence d'une phase solide réactive avec au moins deux récepteurs spécifiques de l'analyte, dont l'un est lié à la phase solide ou se trouve sous une forme susceptible de se lier à la phase solide et un autre porte le groupe marqueur complexe métallique,
(b) on sépare éventuellement la phase solide du liquide d'incubation, et
(c) on décèle la présence et/ou on détermine la quantité de l'analyte dans la phase solide et/ou dans la phase liquide en dosant le marqueur.

10. Procédé selon la revendication 1, caractérisé en ce que l'on effectue le dosage de l'analyte par une analyse par liaison compétitive hétérogène, dans laquelle
(a) on met à incuber l'échantillon liquide contenant l'analyte en présence d'une phase solide réactive avec (al) un récepteur spécifique de l'analyte, qui est lié à la phase solide ou qui se trouve sous une forme liée à la phase solide ou sous une forme susceptible de se lier à la phase solide, et un analogue de l'analyte portant le groupe marqueur complexe métallique, ou avec (a2) un analogue de l'analyte lié à la phase solide ou se trouvant sous une forme susceptible de se lier à la phase solide, et un récepteur spécifique de l'analyte qui porte le groupe marqueur complexe métallique,
(b) on sépare éventuellement la phase solide du liquide d'incubation et
(c) on décèle la présence et/ou on détermine la quantité de l'analyte dans la phase solide et/ou dans la phase liquide en dosant le marqueur.

11. Trousse de réactifs pour la mise en oeuvre d'un procédé selon l'une des revendications 1 à 10, comprenant un complexe métallique luminescent couplé à un constituant spécifique de l'analyte en tant que groupe marqueur, et, comme réactif destiné à éliminer les réactions parasites, un complexe métallique non spécifique qui possède une structure chimiquement apparentée à celle du groupe marqueur.

12. Trousse de réactifs selon la revendication 11, caractérisée en ce que le complexe métallique non spécifique se trouve sous forme du complexe seul, du complexe avec un segment de liaison et/ou du complexe lié à un support.
